# FASCICULE DE BREVET EUROPEEN

(11) **EP 2 001 540 B1**
(45) Date de publication et mention de la délivrance du brevet: **14.11.2018**
(21) Numéro de dépôt: 07731221.3
(22) Date de dépôt: 29.03.2007
(51) Int. Cl.: A61M 16/12, A61M 16/08, A61M 16/04, A61M 16/06

(54) **DISPOSITIF DE RESPIRATION ARTIFICIELLE POUR PATIENTS ATTEINTS D'HYPOXEMIE OU D'ANOXEMIE**
BEATMUNGSGERÄT FÜR PATIENTEN MIT HYPOXÄMIE ODER ANOXÄMIE
ARTIFICIAL RESPIRATION DEVICE FOR PATIENTS SUFFERING FROM HYPOXEMIA OR ANOXEMIA

(30) Priorité: 06.04.2006 FR 0603036
(43) Date de publication de la demande: 17.12.2008
(73) Titulaire: Boussignac, Georges, 92160 Antony (FR)
(72) Inventeur: Boussignac, Georges, 92160 Antony (FR)
(74) Mandataire: Jacobacci Coralis Harle
(86) Numéro de dépôt international: PCT/FR2007/000541
(87) Numéro de publication internationale: WO 2007/118973

(56) Documents cités:
- EP-A1- 0 390 684
- EP-A1- 0 911 051
- WO-A-03/039638
- WO-A-2004/009169
- DE-A1- 2 453 490
- US-A- 5 979 444

## Description

La présente invention concerne un dispositif de respiration artificielle, utilisable sur des patients atteints par des maladies entraînant une hypoxémie ou une anoxémie, telles que arrêt cardiaque, oedème pulmonaire, syndrome respiratoire aigu sévère, grippe aviaire, etc ...

Divers dispositifs d'assistance respiratoire sont déjà connus de l'art antérieur (tels que ceux connus des documents WO 03/039638 A1, EP-0 390 684 et EP-0 911 051).

Par exemple par les brevets européens EP-0 390 684 et EP-0 911 051, on connaît un dispositif d'assistance respiratoire tubulaire, qui forme un canal principal et qui est destiné à être relié par son extrémité distale à une voie respiratoire d'un patient pour que ledit canal principal relie à l'extérieur le système respiratoire dudit patient, ledit dispositif comportant des canaux auxiliaires associés à des moyens de déflexion pour injecter des jets de gaz respiratoire convergents défléchis vers l'intérieur dudit canal principal.

Un tel dispositif d'assistance respiratoire est particulièrement utilisé sur des patients dont la respiration spontanée est insuffisante, lesdits jets de gaz respiratoire défléchis permettant de ventiler lesdits patients. Dans ce cas, le débit de gaz respiratoire utilisé est au maximum de 0,5 litre par minute pour les enfants et de 5 litres par minute pour les adultes.

L'expérience a montré que ce dispositif d'assistance respiratoire connu pouvait être utilisé comme dispositif de respiration artificielle sur des malades atteints d'hypoxémie ou d'anoxémie, à la condition que le débit de gaz respiratoire soit fortement augmenté : par exemple, jusqu'à 5 litres par minute pour les enfants et jusqu'à 50 litres par minute pour les adultes.

Or, avec des débits de gaz respiratoire aussi élevés, la sécurité des malades contre une dilatation excessive (et même un éclatement) des voies respiratoires est difficile à assurer en cas d'obturation accidentelle de l'extrémité proximale dudit canal principal.

Aussi, l'objet de la présente invention est-il de remédier à cet inconvénient en perfectionnant le dispositif d'assistance respiratoire connu, rappelé ci-dessus, pour lui permettre d'être utilisé, en toute sécurité, comme dispositif de respiration artificielle apte à combattre les hypoxémies et les anoxémies.

A cette fin, selon l'invention, le dispositif de respiration artificielle comportant :
- un élément tubulaire qui forme un canal principal et qui est destiné à être relié par son extrémité distale à une voie respiratoire d'un patient alors que l'extrémité proximale dudit élément tubulaire se trouve à l'intérieur dudit patient et que le système respiratoire de celui-ci est relié à l'extérieur par l'intermédiaire dudit canal principal ;
- des canaux auxiliaires qui sont alimentés en gaz respiratoire à débit élevé et qui débouchent dans ledit canal principal ; et
- des moyens de déflexion, pour faire converger les uns vers les autres, à l'intérieur dudit canal principal, les jets de gaz respiratoire injectés par lesdits canaux auxiliaires, afin de former une zone de turbulences dues au débit élevé du gaz respiratoire, est remarquable en ce que ledit élément tubulaire comporte au moins un orifice latéral de sécurité qui traverse sa paroi latérale au moins sensiblement en regard du point de convergence desdits jets de gaz respiratoire et qui est apte à relier à l'extérieur la partie dudit canal principal qui se trouve en aval, par rapport au sens desdits jets de gaz respiratoire, desdits moyens de déflexion, ledit orifice latéral de sécurité permettant l'évacuation des gaz vers l'extérieur en cas de surpression dans la voie respiratoire dudit patient.

En effet, de façon étonnante, l'expérience a montré qu'un tel orifice latéral de sécurité, disposé en regard du point de convergence des jets de gaz respiratoire, ne perturbait pas la ventilation d'un patient à l'aide de ces derniers et, bien entendu, permettait l'évacuation de gaz en cas de surpression dans le système respiratoire dudit patient. Une telle constatation étonnante est probablement due à la formation des turbulences engendrées à la sortie desdits moyens de déflexion par lesdits jets de gaz respiratoire convergents.

Suivant le mode de réalisation du dispositif de respiration artificielle conforme à la présente invention, ladite partie aval du canal principal peut être mise, directement ou indirectement, en communication avec l'extérieur à travers ledit orifice latéral de sécurité. Par exemple :
- dans le cas où le dispositif selon l'invention constitue l'embout d'entrée et de sortie de gaz d'un masque destiné à être appliqué sur le visage d'un patient (ledit embout étant solidaire dudit masque ou rapporté de façon amovible à ce dernier), ledit orifice latéral de sécurité se trouve lui-même à l'extérieur, de sorte qu'il fait communiquer directement la partie aval du canal principal avec l'air libre. Dans ce cas, il est avantageux de masquer les bruits engendrés par lesdits jets de gaz respiratoire et audibles à travers ledit orifice latéral de sécurité, par tout moyen permettant de recouvrir celui-ci, par exemple une enveloppe ou un tampon en matière fibreuse ou poreuse ;
- au contraire, dans le cas où le dispositif selon l'invention est une sonde (nasale ou buccale) destinée à être introduite au moins en partie à l'intérieur d'une voie respiratoire du patient, ledit orifice latéral de sécurité peut se trouver lui-même à l'intérieur de ladite voie respiratoire. Il est alors avantageux de relier ledit orifice latéral de sécurité à l'air libre par l'intermédiaire d'un conduit. Un tel conduit est avantageusement constitué par une gaine souple formant ballon et entourant ledit élément tubulaire, ledit orifice latéral de sécurité débouchant dans ladite gaine qui, elle-même, débouche à l'air libre du côté de l'extrémité proximale de l'élément tubulaire. On remarquera qu'une telle gaine peut protéger les muqueuses du patient contre les frottements avec ladite sonde et sert de plus à amortir au moins partiellement les bruits desdits jets de gaz respiratoire. Toutefois, là encore, il est possible de prévoir tout moyen de matière fibreuse ou poreuse complémentaire pour masquer lesdits bruits.

Pour augmenter encore la sécurité d'utilisation du dispositif de respiration artificielle selon l'invention, celui-ci, quel que soit son mode de réalisation, comporte avantageusement du côté proximal, dans la partie amont (par rapport auxdits jets de gaz respiratoire) dudit canal principal, des obstacles internes saillants tels que des ailettes, empêchant l'obturation hermétique de ladite partie amont par un objet extérieur.

Les figures du dessin annexé feront bien comprendre comment l'invention peut être réalisée. Sur ces figures, des références identiques désignent des éléments semblables.
La figure 1 est une vue schématique, partiellement en coupe axiale, d'un masque respiratoire équipé d'un premier exemple de réalisation du dispositif de respiration artificielle conforme à la présente invention.
La figure 2 montre, à plus grande échelle et en coupe axiale, ledit dispositif de respiration artificielle conforme à l'invention constituant l'embout du masque de la figure 1.
La figure 3 est une vue en bout, selon la flèche III de la figure 2, montrant l'extrémité proximale du dispositif de respiration artificielle des figures 1 et 2.
Les figures 4, 5 et 6 sont des coupes transversales du dispositif de la figure 2, respectivement selon les lignes IV-IV, V-V et VI-VI.
La figure 7 montre, en coupe horizontale axiale à grande échelle, un second exemple de réalisation du dispositif de respiration artificielle conforme à la présente invention.

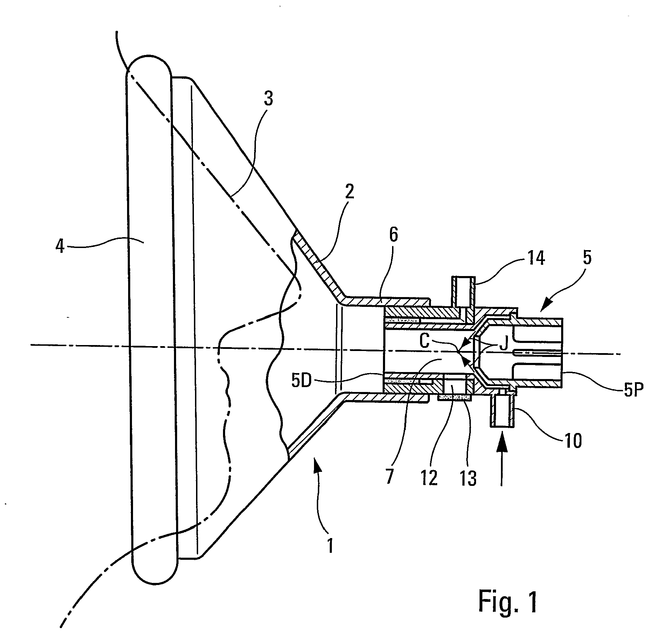

Le masque respiratoire 1, représenté sur la figure 1, comporte une coque rigide de forme générale tronconique 2, pouvant être appliquée sur le visage 3 d'un patient par l'intermédiaire d'un coussinet 4, bordant son ouverture périphérique. Du côté opposé, ledit masque 1 est pourvu d'un dispositif de respiration artificielle conforme à la présente invention, comportant un élément tubulaire 5, fixe ou emboîté sur une saillie tubulaire 6 de ladite coque 2. L'élément tubulaire 5 sert d'embout d'entrée et de sortie de gaz dans le masque 1, son extrémité proximale 5P étant à l'air libre, alors que son extrémité distale 5D se trouve du côté du masque 1.

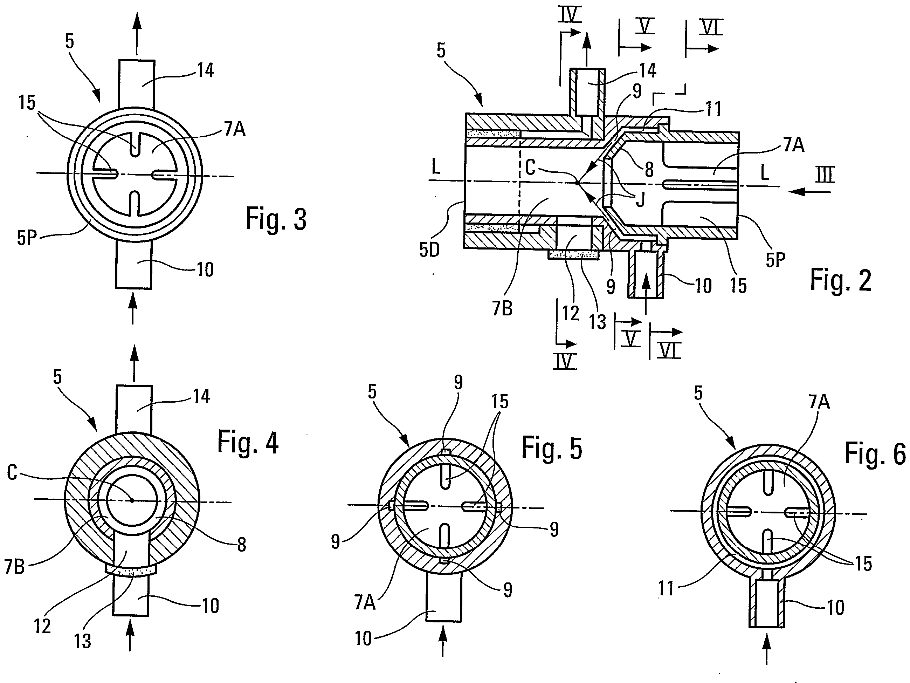

Comme le montrent plus en détail les figures 2 à 6, l'élément tubulaire 5 forme un canal principal interne 7 et il comporte, en partie médiane, des moyens de déflexion 8, dirigés vers l'axe L-L dudit canal 7. Les moyens de déflexion 8 ont pour objet de défléchir, en direction dudit axe du canal principal 7, des jets de gaz respiratoire J injectés par des canaux auxiliaires périphériques 9, alimentés à partir d'un embout d'amenée 10, par l'intermédiaire d'une chambre annulaire périphérique 11, lesdits jets de gaz respiratoire convergeant ainsi vers un point de convergence C de l'axe L-L dudit canal principal 7.

Lesdits moyens de déflexion 8 partagent donc le canal principal 7 en une partie amont 7A qui est disposée du côté de l'extrémité proximale 5P de l'élément tubulaire 5 et en une partie aval 7B, dans laquelle se trouve le point de convergence C et qui est disposée du côté de l'extrémité distale 5D dudit élément tubulaire 5.

Selon la particularité principale de la présente invention, la paroi latérale dudit élément tubulaire 5 est percée par un orifice latéral de sécurité 12, disposé en regard du point de convergence C des jets de gaz respiratoire J et apte à mettre directement à l'air libre la partie aval 7B du canal principal 7.

Un tampon fibreux ou poreux 13 recouvre l'orifice latéral de sécurité 12 pour éviter la propagation vers l'extérieur, à travers ledit orifice, du bruit engendré par les jets de gaz respiratoire J.

Par ailleurs, l'élément tubulaire 5 comporte un embout 14 pour le prélèvement de gaz et/ou la mesure de pression.

Comme cela est illustré par les figures, dans la partie amont 7A du canal principal 7, on a prévu des obstacles, par exemple des ailettes convergentes 15, empêchant l'introduction accidentelle d'un objet apte à obturer hermétiquement ladite partie amont 7A.

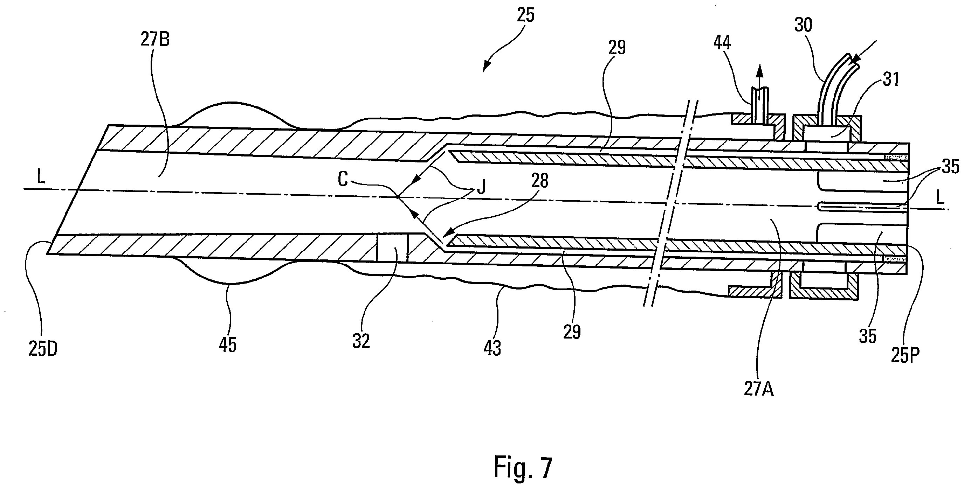

La variante de réalisation du dispositif de respiration artificielle selon l'invention, représenté sur la figure 7, comporte un élément tubulaire 25 souple, apte à former une sonde nasale ou buccale. L'élément tubulaire 25 forme un canal principal interne 27 et il comporte des moyens de déflexion 28, dirigés vers l'axe L-L dudit canal 27. Les moyens de déflexion 28 ont pour objet de défléchir, en direction dudit axe L-L, des jets de gaz respiratoire J injectés par des canaux auxiliaires 29, alimentés à partir d'un conduit d'amenée 30, par l'intermédiaire d'une chambre périphérique 31, lesdits jets de gaz respiratoire J convergeant ainsi vers un point de convergence C dudit axe L-L.

Les moyens de déflexion 28 partagent le canal principal 27 en une partie amont 27A qui est disposée du côté de l'extrémité proximale 25P de l'élément tubulaire 25 et en une partie aval 27B, dans laquelle se trouve le point de convergence C et qui est disposé du côté de l'extrémité distale 25D de l'élément tubulaire 25.

Dans la paroi latérale de l'élément tubulaire 25 est percé un orifice latéral de sécurité 32, disposé en regard du point de convergence C des jets de gaz respiratoire J.

On remarquera que, sur la figure 7, les éléments 25 à 31 correspondent respectivement aux éléments 5 à 11 des figures 1 à 6.

L'élément tubulaire 25 est, sur la plus grande partie de sa longueur, enveloppé par une gaine étanche souple 43, du type connu ballon gonflable, dans laquelle débouche ledit orifice latéral de sécurité 32 et qui est elle-même en communication avec l'air libre par un embout 44, disposé du côté proximal de l'embout tubulaire 25. Ainsi, l'orifice 32 est apte à mettre la partie aval 27B du canal 27 à l'air libre, à travers ledit ballon 43 et l'embout 44.

Dans la partie amont 27A du canal principal 7, sont disposés des obstacles, par exemple des ailettes convergentes 35, empêchant l'introduction accidentelle d'un objet apte à obturer hermétiquement ladite partie amont 27A.

De plus, de façon connue, l'élément tubulaire 25 peut comporter un ballon de fixation gonflable 45, en aval de la gaine 43.

Un tel ballon 45 n'est pas recommandé pour les sondes pédiatriques. De plus, notamment dans ce dernier cas, il est préférable que, en fonctionnement, l'extrémité distale 25D se trouve en avant des cordes vocales du patient.

De préférence, lesdites ailettes 15 ou 35 interdisent l'accès à au moins 70% de la section desdites parties amont 7A ou 27A.

## Revendications

1. Dispositif de respiration artificielle comportant :
- un élément tubulaire (5, 25) qui forme un canal principal (7, 27) et qui est destiné à être relié par son extrémité distale (5D, 25D) à une voie respiratoire d'un patient (3) alors que l'extrémité proximale (5P, 25P) dudit élément tubulaire se trouve à l'extérieur dudit patient et que le système respiratoire de celui-ci est relié à l'extérieur par l'intermédiaire dudit canal principal (7, 27) ;
- des canaux auxiliaires (9, 29) qui sont alimentés en gaz respiratoire à débit élevé et qui débouchent dans ledit canal principal (7, 27) ; et
- des moyens de déflexion (8, 28), pour faire converger les uns vers les autres, à l'intérieur dudit canal principal (7, 27), les jets de gaz respiratoire (J) injectés par lesdits canaux auxiliaires (9, 29), afin de former une zone de turbulences dues au débit élevé du gaz respiratoire,
**caractérisé en ce que** ledit élément tubulaire (5, 25) comporte au moins un orifice latéral de sécurité (12, 32) qui traverse sa paroi latérale au moins sensiblement en regard du point de convergence (C) desdits jets de gaz respiratoire (J) et qui est apte à relier à l'extérieur la partie (7B, 27B) dudit canal principal (7, 27) qui se trouve en aval, par rapport au sens desdits jets de gaz respiratoire, desdits moyens de déflexion (8, 28), ledit orifice latéral de sécurité permettant l'évacuation des gaz vers l'extérieur en cas de surpression dans la voie respiratoire dudit patient.

2. Dispositif de respiration artificielle selon la revendication 1, **caractérisé en ce que** ladite partie aval (7B, 27B) dudit canal principal est en communication directe avec l'extérieur à travers ledit orifice latéral de sécurité (12).

3. Dispositif de respiration artificielle selon la revendication 2, **caractérisé en ce qu'**il comporte des moyens fibreux ou poreux (13) pour masquer le bruit desdits jets de gaz respiratoire traversant ledit orifice latéral de sécurité (12).

4. Dispositif de respiration artificielle selon la revendication 1, **caractérisé en ce qu'**il comporte un conduit (43) reliant ledit orifice latéral de sécurité (32) à l'extérieur.

5. Dispositif de respiration artificielle selon la revendication 4, **caractérisé en ce que** ledit conduit est constitué par une gaine souple (43), entourant ledit élément tubulaire (25) et débouchant à l'extérieur du côté de ladite extrémité proximale (25P) de ce dernier.

6. Dispositif de respiration artificielle selon l'une des revendications 1 à 5, **caractérisé en ce que** la partie amont (7A, 27A) dudit canal principal (7, 27) comporte des obstacles internes saillants (15, 35) empêchant l'obturation hermétique de ladite partie amont (7A, 27A).

## Patentansprüche

1. Beatmungsgerät mit
- einem rohrförmigen Element (5, 25), das einen Hauptkanal (7, 27) bildet und das dazu bestimmt ist, mit seinem distalen Ende (5D, 25D) an einem Atemweg eines Patienten (3) angeschlossen zu werden, während sich das proximale Ende (5P, 25P) des rohrförmigen Elements außerhalb des Patienten befindet und das Atmungssystem desselben durch den Hauptkanal (7, 27) nach außen verbunden ist,
- Hilfskanälen (9, 29), die mit Atemgas mit hohem Durchsatz versorgt werden und die in den Hauptkanal (7, 27) münden, und
- Umlenkmitteln (8, 28), um im Inneren des Hauptkanals (7, 27) die über die Hilfskanäle (9, 29) eingespritzten Atemgasstrahlen (J) aufeinander zu zu führen, um eine durch den hohen Durchsatz an Atemgas bedingte Turbulenzzone zu schaffen,
**dadurch gekennzeichnet, daß** das rohrförmige Element (5, 25) mindestens ein seitliches Sicherheitsloch (12, 32) aufweist, das seine seitliche Wandung wenigstens im Wesentlichen gegenüber dem Konvergenzpunkt (C) der Atemgasstrahlen (J) durchquert und das dazu ausgelegt ist, den Teil (7B, 27B) des Hauptkanals (7, 27) nach außen zu verbinden, der sich in Bezug auf die Richtung der Atemgasstrahlen stromabwärts von den Umlenkmitteln (8, 28) befindet, wobei das seitliche Sicherheitsloch im Fall eines Überdrucks im Atemweg des Patienten den Abfluß der Gase nach außen ermöglicht.

2. Beatmungsgerät gemäß Anspruch 1, **dadurch gekennzeichnet, daß** der stromabwärts liegende Teil (7B, 27B) des Hauptkanals durch das seitliche Sicherheitsloch (12) in direkter Verbindung mit dem Freien steht.

3. Beatmungsgerät gemäß Anspruch 2, **dadurch gekennzeichnet, daß** es faserartige oder poröse Mittel (13) zum Verdecken der Geräusche der das seitliche Sicherheitsloch (12) durchströmenden Atemgase aufweist.

4. Beatmungsgerät gemäß Anspruch 1, **dadurch gekennzeichnet, daß** es eine Leitung (43) aufweist, die das seitliche Sicherheitsloch (32) nach außen verbindet.

5. Beatmungsgerät gemäß Anspruch 4, **dadurch gekennzeichnet, daß** die Leitung aus einem flexiblen Schlauch (43) besteht, der das rohrförmige Element (25) umgibt und auf der Seite des proximalen Endes (25P) des letzteren nach außen mündet.

6. Beatmungsgerät gemäß einem der Ansprüche 1 bis 5, **dadurch gekennzeichnet, daß** der stromaufwärts liegende Teil (7A, 27A) des Hauptkanals (7, 27) innere hervorstehende Hindernisse aufweist, die ein hermetisches Verschließen des stromaufwärts liegenden Teils (7A, 27A) verhindern.

## Claims

1. An artificial respiration device comprising:
- a tubular element (5, 25) which forms a main channel (7, 27) and which is designed to be connected via its distal end (5D, 25D) to an airway of a patient (3), while the proximal end (SP, 25P) of said tubular element is located outside said patient and the latter's respiratory system is connected to the outside by way of said main channel (7, 27);
- auxiliary channels (9, 29) which are supplied with high flow respiratory gas and that open into said main channel (7, 27); and
- deflecting means (8, 28) which ensure that the jets of respiratory gas (J) injected via said auxiliary channels (9, 29) are caused to converge on each other inside said main channel (7, 27), in order to create a turbulence zone due to the high flow of the respiratory gas;
**characterized in that** said tubular element (5, 25) comprises at least one lateral safety orifice (12, 32), which extends through its lateral wall at least substantially opposite the point of convergence (C) of said jets of respiratory gas (J) and is able to connect to the outside that part (7B, 27B) of said main channel (7, 27) located downstream in relation to the direction of said jets of respiratory gas from said deflecting means (8, 28), said lateral safety orifice allowing the evacuation of gas in the event of excess pressure in the patient's airway.

2. The artificial respiration device as claimed in claim 1, **characterized in that** said downstream part (7B, 27B) of said main channel is in direct communication with the outside by way of said lateral safety orifice (12).

3. The artificial respiration device as claimed in claim 2, **characterized in that** it comprises fibrous or porous means (13) for masking the noise of said jets of respiratory gas passing through said lateral safety orifice (12).

4. The artificial respiration device as claimed in claim 1, **characterized in that** it comprises a conduit (43) connecting said lateral safety orifice (32) to the outside.

5. The artificial respiration device as claimed in claim 4, **characterized in that** said conduit is formed by a flexible sheath (43) surrounding said tubular element (25) and opening to the outside at said proximal end (25P) of the latter.

6. The artificial respiration device as claimed in claim 1, **characterized in that** the upstream part (7A, 27A) of said main channel (7, 27) comprises protruding internal obstacles (15, 35) that prevent hermetic closure of said upstream part (7A, 27A).
